Europäisches Patentamt

European Patent Office

Office européen des brevets

(19)

(11) Veröffentlichungsnummer: **0 019 720**
**B1**

(12) **EUROPÄISCHE PATENTSCHRIFT**

(45) Veröffentlichungstag der Patentschrift: **08.02.84**

(21) Anmeldenummer: **80102219.5**

(22) Anmeldetag: **25.04.80**

(51) Int. Cl.³: **A 61 K 7/06**, A 61 K 7/48, A 61 K 31/385

(54) Mesulfen-Zubereitungen und Verfahren zu ihrer Herstellung.

(30) Priorität: **28.05.79 IT 2305079**

(43) Veröffentlichungstag der Anmeldung:
**10.12.80 Patentblatt 80/25**

(45) Bekanntmachung des Hinweises auf die Patenterteilung:
**08.02.84 Patentblatt 84/6**

(84) Benannte Vertragsstaaten:
**BE DE FR GB NL**

(56) Entgegenhaltungen:
CH - A - 241 597
CH - A - 246 914

UNLISTED DRUGS, vol. 20, Nr. 6, Juni 1968,
Chathan N.J. USA "SOUFROL", Seite 91, item h

THE MERCK INDEX, 8th edition, Merck & Co
Rahway, N.J. USA "Mesulphen", Seite 665, linke
Spalte - rechte Spalte

Die Akte enthält technische Angaben, die nach
dem Eingang der Anmeldung eingereicht wurden
und die nicht in dieser Patentschrift enthalten
sind.

(73) Patentinhaber: **BAYER ITALIA S.p.A.**
**Viale Certosa 126**
**I-20156 Mailand (IT)**

(72) Erfinder: **Dondi, Gilberto**
**viale dei Tigli 13**
**I-20095 Cusano Milanino (IT)**
Erfinder: **Sacchi, Giovanni**
**via dei Cignoli 3**
**I-20151 Mailand (IT)**

(74) Vertreter: **Klausner, Erich**
**c/o Ufficio Internazionale Brevetti Ing.C. Gregorj**
**Via Dogana 1**
**I-20100 Mailand (IT)**

(56) Entgegenhaltungen:
**CHEMICAL ABSTRACTS, Band 39, No.1, 10-01-
1956, Zusammenfassung 365-7 Columbus,
Ohio, US R.M. GORDON et al.: "The lethal action
of benzyl benzoate dimethylthianthrene (Mitigal)
and tetra-ethylthiuram monosulfide (Tetmos) on
the scabies-producing mites, notoedres Sp. and
sarcoptes scabiei var. hominis, when tested in
vitro"**

Courier Press, Leamington Spa, England.

# 0 019 720

Mesulfen-Zubereitungen und Verfahren zu ihrer Herstellung

Die vorliegende Erfindung betrifft Mesulfen Zubereitungen, und Verfahren zu ihrer Herstellung. Die Mittel eignen sich zur Bekämpfung von Ungeziefer an insbesondere behaarten Körper teilen sowie zur Behandlung von Seborrhoe und Juckreiz.

Mesulfen, (=2,7 Dimethyltioantrene) wird seit langem wegen seiner besonderen antiparasitären Eigenschaften und wegen seiner juckreizunterbindenden Wirkung zur Bekämpfung von Ungeziefer beim Menschen und bei Tieren eingesetzt.

Die Wirkung gegen Pedikulose konnte wegen der Anwendungsschwierigkeiten in den behaarten Zonen und vor allem im Haar nie vollständig ausgenützt werden. Da der Wirkstoff wie bekannt in Form einer sehr dicken Flüssigkeit von hoher Viskosität und Schmierigkeit vorhanden ist, ist seine praktische Anwendung fast unmöglich. Zu dem kommt der sehr unangenehme Nachteil eines charakteristischen, widerlichen und anhaltenden Geruchs, der den Wirkstoff unanbietbar macht.

Aus Beispiel 5 der CH—A—241597 sind Mischungen, die Mesulfen neben oberflächenaktiven Mitteln enthalten zur Schädlingsbekämpfung bekannt, diese Mischungen enthalten Xylol als Lösungsmittel. Wie bekannt, können Zubereitungen, die aromatische Lösungsmittel enthalten, nicht für die Körperhygiene wegen deren Toxizität eingesetzt werden.

Auch können ölige Gemische oder Emulsionen, die z.B. aus Unlisted Drugs (Vol. 20, Nr. 6, Juni 1968, Chatan, N. J. USA, "Soufrol", Seite 91, item h), oder aus Helwig, Moderne Arzneimittel (5. Auflage, S. 1055 "Liquidin®-Schwefel") bekannt sind, nicht auf die behaarten Zonen der Körpers appliziert werden, denn, wie z.B. aus Frederick H. Meyers et al. Review of Medical Pharmacology (7th edition, Lange Medical Publications, Los Altos, California, USA, 1980 S. 35), bekannt ist, können solche Applizierungen zu Follicolitis führen.

Wie aus Moderne Auzneimittel, (siehe oben) zu entnehmen ist, hat Liquidin®-Schwefel auch weitere Kontraindikationen. Aufgabe der Erfindung ist es daher, neue Zubereitungen zur Bekämpfung von Ungeziefer, Seborrhoe un Juckreiz bereitzustellen, die eine rationelle Anwendung des Mesulfens in den haarigen Zonen und vor allem im Haar ermöglichen.

Eine weitere Aufgabe der Erfindung ist es, Verfahren zur Herstellung dieser neuen Zubereitungen mit Wirkung gegen Ungeziefer, Seborrhoe und Juckreiz bereitzustellen.

Die Lösung dieser Aufgabe erfolgt durch Bereitstellung von Zubereitungen gegen Ungeziefer, Seborrhoe und Juckreiz, die durch folgende zusammensetzung (jeweils Gew.-%) gekennzeichnet sind:

| | |
|---|---|
| Mesulfen | 4—8% |
| Glyzerinpolyglykoläterrizinoleat | 6—8% |
| Laurylpolyäthylenglykoläther Natriumsulfat 50% | 40% |
| Natrium Laurylsarkosinat | 15% |
| Aethoxyliertes Lanolin | 3% |
| Propylenglkol | 1% |
| Aethylendiamintetraessigsäure Dinatrium salz | 0,2% |
| Benzylalkohol | 1% |
| Parfüm | 0,4% |
| Wasser (gereinigt) | zu 100% |

sowie gegebenenfalls weiteren in der Kosmetik üblichen Hilfsmitteln und/oder Zusatzstoffen.

Es wurde nun gefunden, dass, wenn man Mesulfen in Form eines Shampoos zubereitet, durch die Trägerwirkung der in ihm enthaltenen oberflächenaktiven Substanzen eine gleichmässige Verteilung desselben erreicht wird, das sich kapillarisch in jedem kleinsten Teil der zu behandelnden Zonen ausbreitend, eine Anwendung in Form kleinerer Konzentrationen, als es die herkömmlicherweise üblichen sind, möglich macht. Ausserdem erzielt man dank der dem Shampoo eigenen reinigenden Wirkung eine sorgfältige Hygiene der Kopfhaut, die nicht nur das sich Festsetzen von Ungeziefer verhinden kann, sondern auch seine endgültige Beseitigung begünstigt.

Zur Herstellung des Shampoos wird zunächst eine Vermischung von Mesulfen mit den oberflächenaktiven Substanzen hergestellt, die man anschließend mit der Lösung der Schaumbildner, Schaumstabilisatoren, Rückfettungszusätze, Komplexbildner, Bakterienbekämpfungsmittel vereinigt.

Die Erfindung wird durch folgende Beispiele näher erläutert:

2

# 0 019 720

Beispiele 1—3
(Shampoo)

Ansatz:

| | | | |
|---|---|---|---|
| Mesulfen | 4,0g | 6,0g | 8,0g |
| Glyzerinpolyglykolätherrizinoleat | 6,0g | 4,0g | 8,0g |
| Laurylpolyäthylenglykoläther Natrium Sulfat 50% | 40,0g | 40,0g | 40,0g |
| Natrium Laurylsarkosinat | 15,0g | 15,0g | 15,0g |
| Aethoxyliertes Lanolin | 3,0g | 3,0g | 3,0g |
| Propylenglykol | 1,0g | 1,0g | 1,0g |
| Aethylendiamintetraessigsäure Dinatrium salz | 0,2g | 0,2g | 0,2g |
| Benzylalkohol | 1,0g | 1,0g | 1,0g |
| Parfüm | 0,4g | 0,4g | 0,4g |
| Wasser (gereinigt) | zu 100g | 100g | 100g |

**Patentansprüche**

1. Zubereitung gegen Ungeziefer, Seborrhoe und Juckreiz, gekennzeichnet, durch folgende Zusammensetzung (jeweils Gew.-%):

| | |
|---|---|
| Mesulfen | 4—8% |
| Glyzerinpolyglykoläterrizinoleat | 6—8% |
| Laurylpolyäthylenglykoläther Natriumsulfat 50% | 40% |
| Natrium Laurylsarkosinat | 15% |
| Aethoxyliertes Lanolin | 3% |
| Propylenglykol | 1% |
| Aethylendiamintetraessigsäure Dinatrium salz | 0,2% |
| Benzylalkohol | 1% |
| Parfüm | 0,4% |
| Wasser (gereinigt) | zu 100% |

sowie gegebenenfalls weiteren in der Kosmetik üblichen Hilfsmitteln und/oder Zusatzstoffen.

2. Verfahren zur Herstellung von Zubereitungen gemäss Anspruch 1, dadurch gekennzeichnet, dass man zunächst eine Vormischung einer wirksamen Menge Mesulfen mit den oberflächenaktiven Substanzen herstellt und dass man diese Vormischung anschließend mit der Lösung der weiteren verwendeten Zusätzen vereinigt.

**Revendications**

1. Préparation contre les parasites, la séborrhée et le prurit, caractérisée par la composition suivante (chaque fois en pourcentage en poids):

| | |
|---|---|
| Mésulfène | 4 à 8% |
| Ricinoléate de glycérylpolyglycoléther | 6 à 8% |
| Sulfat de sodium et de laurylpolyéthylène-glycoléther à 50% | 40% |
| Laurylsarcosinate de sodium | 15% |
| Lanoline éthoxylée | 3% |
| Propylène-glycol | 1% |
| Sel di-sodique de l'acide éthylènediamine tétraacétique | 0,2% |
| Alcool benzylique | 1% |
| Parfum | 0,4% |
| Eau (purifiée) pour compléter à | 100% |

et, éventuellement d'autres adjuvants et/ou additifs usuels en cosmétique.

2. Procédé de fabrication de préparations selon la revendication 1, caractérisé en ce qu'on prépare tout d'abord un mélange préliminaire d'une quantité active de mésulfène avec les substances tensio-actives et l'on réunit ensuite ce mélange préliminaire avec la solution des autres additifs utilisés.

**Claims**

1. A preparation against parasites, seborrhea and pruritus, characterized by the fact of having the following composition (parts in weight-%):

| | |
|---|---|
| Mesulphen | 4—8 |
| Glycerol polyglycol ether ricineolate | 6—8 |
| Lauryl polyethyleneglycol ether sodium sulfate | 40 |
| Sodium lauryl sarcosinate | 15 |
| Ethoxylated lanoline | 3 |
| Propylene glycol | 1 |
| Ethylenediaminetetracetic acid | 0.2 |
| Benzyl alcohol | 1 |
| Perfume | 0.4 |
| Water (purified) | up to 100 |

as well as, possibly, other adjuvants and/or auxiliaries commonly used in the cosmetic industry.

2. A process for making a preparation according to claim 1 characterized by the fact that a pre-mix of an efficient amount of mesulphen is first prepared with a surface-active agent(s) and that said pre-mix is then combined with the solution of the other components used.